(19) Europäisches Patentamt
European Patent Office
Office européen des brevets

(11) EP 0 875 495 B1

(12) FASCICULE DE BREVET EUROPEEN

(45) Date de publication et mention de la délivrance du brevet:
**21.06.2000 Bulletin 2000/25**

(51) Int Cl.[7]: **C07C 65/40**, C07C 51/41, A61K 31/19, A61K 7/48

(21) Numéro de dépôt: **98400946.4**

(22) Date de dépôt: **16.04.1998**

(54) **Nouveaux dérivés de l'acide salicylique et leur utilisation dans une composition cosmétique et/ou dermatologique**

Neue Salicylsäurederivate und deren Verwendung in kosmetischen und/oder dermatologischen Zusammensetzungen

New derivatives of salicylic acid and their use in cosmetic and/or dermatological compositions

(84) Etats contractants désignés:
**AT BE CH DE ES FR GB IT LI NL SE**

(30) Priorité: **30.04.1997 FR 9705412**

(43) Date de publication de la demande:
**04.11.1998 Bulletin 1998/45**

(73) Titulaire: **L'OREAL**
**75008 Paris (FR)**

(72) Inventeurs:
- **Philippe, Michel**
  **91320 Wissous (FR)**
- **Cohen, Catherine**
  **75012 Paris (FR)**

(74) Mandataire: **Tezier Herman, Béatrice**
**L'OREAL-DPI**
**6 rue Bertrand Sincholle**
**92585 Clichy Cédex (FR)**

(56) Documents cités:
**FR-A- 2 053 011** **FR-A- 2 581 542**
**US-A- 1 933 520** **US-A- 2 431 127**

- **BIALOBRZESKI, M. ; NENCKI, M.: "ÜBER DIE ACETSALICYLSÄURE" BERICHTE DER DEUTSCHEN CHEMISCHEN GESELLSCHAFT, vol. 30, 1897, pages 1776-1779, XP002075249**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention se rapporte à de nouveaux dérivés de l'acide salicylique, à leur procédé de préparation et à leur utilisation dans les domaines cosmétique et/ou dermatologique. Ces dérivés peuvent être utilisés aussi dans le domaine pharmaceutique ou vétérinaire. Plus spécialement, ces dérivés sont utilisés comme agents exfoliants, notamment dans une composition destinée au soin et/ou au maquillage de la peau, des muqueuses et/ou des fibres kératiniques d'être humain, en particulier pour lutter contre les signes du vieillissement cutané et/ou pour améliorer l'éclat du teint et/ou pour lisser la peau du visage et/ou du corps et/ou pour traiter les rides et les ridules de la peau et/ou pour stimuler le processus de renouvellement épidermique et/ou pour traiter l'acné et/ou les désordres cutanés.

Il est connu d'utiliser des dérivés de l'acide salicylique comme agent kératolytique pour traiter l'acné et comme agent d'antivieillissement dans des compositions cosmétiques et/ou dermatologiques. Ainsi, les documents FR-A-2 581 542 et EP-A-378 936 décrivent de tels dérivés.

Les dérivés d'acide salicylique sont d'un grand intérêt, étant donné leurs effets biologiques sur la peau, notamment sur les principaux signes cliniques du vieillissement cutané que sont les ridules et rides, la désorganisation du "grain" de la peau, la modification du teint de la peau et la perte de fermeté et de tonicité de la peau. Cependant, l'utilisation de ces dérivés pose un problème dans la mesure où ils peuvent provoquer des picotements, des démangeaisons, des tiraillements après leur application, pouvant conduire à un important inconfort. L'utilisation de ces composés pour les utilisateurs à peau sensible est donc souvent rédhibitoire.

Les documents WO-A-96/19184, WO-A-96/19182 et WO-A-96/19228 décrivent l'utilisation de sels divalents de strontium, manganèse, magnésium et calcium, solubles dans l'eau pour diminuer l'irritation de composés ayant un effet irritant, notamment d'exfoliants tels que les hydroxy-acides. Toutefois, dans ces documents, l'actif irritant reste dans la composition sous forme acide.

Par ailleurs, le document EP-A-413 528 décrit l'utilisation de composés amphotères pour diminuer l'irritation des hydroxy-acides. Selon ce document, l'utilisation de sels inorganiques d'hydroxy-acides diminuent l'efficacité de ces derniers.

La demanderesse a découvert de façon surprenante de nouveaux dérivés salifiés divalents de l'acide salicylique permettant de diminuer l'irritation induite par ces acides. En outre, contrairement à l'enseignement du document EP-A-413 528, la demanderesse a trouvé que ces dérivés de l'acide salicylique avaient des propriétés au moins égales voire supérieures à celles de l'acide correspondant sous forme libre.

L'invention a donc pour objet un dérivé salifié de formule (I) :

$$AR^{-}\ S^{2+}\ AR^{-} \qquad (I)$$

dans laquelle :

$S^{2+}$ représente un cation inorganique divalent ;

$AR^{-}$ représente le radical de formule (II) :

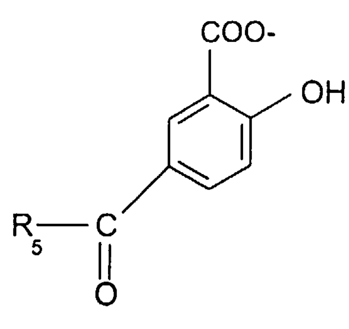

(II)

dans laquelle :

$R_5$ représente une chaîne aliphatique linéaire ou ramifiée, saturée ou insaturée, ayant de 3 à 15 atomes de carbone, préférentiellement de 7 à 11 atomes de carbone. Le composé de l'invention présente la particularité par rapport

aux sels de l'art antérieur d'être lipophile.

De façon avantageuse, les dérivés de l'acide salicylique sont choisis parmi les sels des acides n-octanoyl-5-salicylique, n-décanoyl-5-salicylique, n-dodécanoyl-5-salicylique. On peut toutefois utiliser les sels de l'acide propanoyl-5 salicylique.

Les cations inorganiques divalents sont en particulier ceux des éléments des colonnes IB, IIA, IIB, IIIB, IIIA, IVB, VB, VIB, VIIB, VIII de la classification périodique (version CAS). Les cations divalents préférés sont ceux des colonnes IB, IIA, IIB, VIIB, VIII et encore mieux ceux des colonnes IIA, IIB et VIIB de la classification périodique et notamment ceux de strontium, de calcium, de magnésium, de baryum et de manganèse.

Les dérivés préférés selon l'invention sont le sel de strontium de l'acide 5-octanoylsalicylique, le sel de calcium de l'acide 5-octanoyl-salicylique, le sel de magnésium de l'acide 5-octanoyl-salicylique.

L'invention a aussi pour objet un procédé de fabrication du dérivé salifié de formule (I), consistant à faire réagir un dérivé de l'acide salicylique de formule (III) :

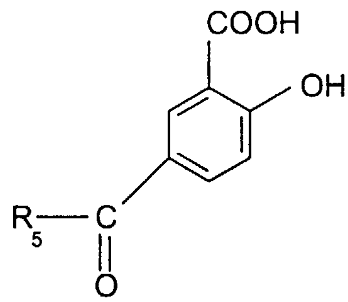

(III)

dans laquelle :

$R_5$ représente une chaîne aliphatique, linéaire ou ramifiée, saturée ou insaturée, ayant de 3 à 15 atomes de carbone, avec un composé comportant un cation inorganique divalent en présence d'un solvant.

Comme solvants, on peut utiliser les alcools comportant de 1 à 12 atomes de carbone, et par exemple l'isopropanol et le butanol, les hydrocarbures tels que l'hexane et l'heptane, les cétones telles que la méthyléthylcétone, l'eau et de façon plus générale, tout composé susceptible de solvater l'acide et le composé comportant le cation inorganique, comme les éthers et les amines..

Le composé comportant le cation inorganique peut être choisi parmi les carbonates, bicarbonates, sulfates, glycérophosphates, borates, chlorures, nitrates, acétates, hydroxydes, persulfates, les sels d'$\alpha$-hydroxy-acides (citrates, tartrates, lactates, malates) ou d'acides de fruits, les sels d'acides aminés (aspartate, arginate, glycocholate, fumarate), les sels d'acides gras (palmitate, oléate, caséinate, béhénate) et tout autre composé susceptible de libérer un cation divalent. De façon avantageuse, on utilise un carbonate ou un hydroxyde.

L'invention a aussi pour objet une composition comprenant au moins un dérivé salifié tel que défini précédemment. Cette composition est en particulier une composition topique, en particulier cosmétique et/ou dermatologique. Dans ce cas, elle contient un milieu physiologiquement acceptable. Par milieu physiologiquement acceptable, on entend un milieu compatible avec la peau, les muqueuses (y compris l'intérieur des paupières et les lèvres), les ongles et/ou les fibres kératiniques (cheveux et cils).

Les dérivés selon l'invention doivent être présents en une quantité efficace pour assurer le résultat escompté. Ils peuvent être par exemple présents dans une composition selon l'invention en une quantité allant de 0,001 à 30 %, de préférence de 0,01 à 20 % et mieux de 0,1 à 10 % du poids total de la composition.

Les compositions contenant un dérivé selon l'invention peuvent se présenter sous toutes les formes galéniques normalement utilisées et notamment celles appropriées pour une application topique, par exemple sous forme d'une solution aqueuse, alcoolique, hydro-alcoolique ou huileuse, d'un gel aqueux ou huileux, d'un produit anhydre liquide, pâteux ou solide, d'une émulsion du type eau-dans-huile (E/H), huile-dans-eau (H/E) ou multiple (E/H/E ou H/E/H), d'une microémulsion ou d'une dispersion de vésicules de type ionique et/ou non ionique. Ces compositions sont préparées selon les méthodes usuelles.

Ces compositions peuvent être plus ou moins fluides et avoir l'aspect d'une crème blanche ou colorée, d'une pommade, d'un lait, d'une lotion, d'un sérum, d'une pâte, d'une mousse. Elles peuvent éventuellement être appliquées sur la peau sous forme d'aérosol. Elles peuvent également se présenter sous forme solide, et par exemple sous forme de stick.

Ces compositions peuvent comprendre au moins un adjuvant choisi parmi l'eau, les corps gras, les conservateurs, les gélifiants, les tensioactifs et émulsionnants, les antioxydants, les charges, les solvants, les parfums, les

matières colorantes, les filtres, les actifs cosmétiques et/ou dermatologiques tels que les hydratants, les vitamines et les actifs antivieillissement autres que les dérivés de formule (I), et leurs mélanges. Les quantités utilisées de ces différents additifs sont celles classiquement utilisées dans le domaine cosmétique et/ou dermatologique.

Les corps gras peuvent être choisis parmi les huiles de synthèse, les huiles d'origine animale, les huiles d'origine végétale, les huiles minérales (huile de vaseline), les huiles de silicone, les huiles fluorées et leurs mélanges. On peut aussi utiliser des alcools gras, des acides gras, des cires.

Lorsque la composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 20 % en poids par rapport au poids total de la composition.

Lorsque la composition est une solution ou un gel huileux, la phase grasse peut représenter plus de 90 % du poids total de la composition.

Comme émulsionnants utilisables dans l'invention, on peut citer par exemple les éthers oléiques de polyéthylène glycol tels que les produits vendus sous les dénominations BRIJ92 et BRIJ96 par la société ICI.

Les quantités des différents constituants des compositions selon l'invention sont celles classiquement utilisées dans les domaines considérés. La nature des adjuvants et leurs quantités doivent être telles qu'elles ne modifient pas les propriétés des dérivés selon l'invention.

Les compositions de l'invention peuvent être utilisées comme produit de nettoyage, de protection, de traitement ou de soin et/ou comme produit de maquillage de la peau du visage et/ou du corps, des muqueuses et/ou des fibres kératiniques. Elles constituent notamment des crèmes de traitement ou de soin pour le visage, pour les mains ou pour le corps, (par exemple crèmes de jour, crèmes de nuit, crèmes solaires), des laits corporels de protection ou de soin, des lotions (lotions de nettoyage, lotions solaires), des gels ou mousses pour le soin de la peau, des compositions pour le bain. Elles constituent aussi des produits de maquillage notamment des joues, des lèvres, des cils et des paupières, tels que des fonds de teint, des rouges à lèvre et des fards à paupière : à cet effet, elles peuvent comprendre des matières colorantes et notamment des pigments ou des colorants chimiques.

Elles peuvent être également utilisées pour les cheveux sous forme de solutions aqueuses, alcooliques ou hydroalcooliques, ou sous forme de crèmes, de gels, d'émulsions, ou de mousses et notamment comme compositions pour soins capillaires, tels que shampooings, lotions traitantes, lotions restructurantes pour les cheveux, lotions ou gels antichute, shampooings antiparasitaires, etc

Les compositions selon l'invention peuvent également consister en des préparations solides constituant des savons ou des pains de nettoyage.

Comme le montre le test décrit ci-dessous, les dérivés selon l'invention ont de bonnes propriétés exfoliantes.

L'invention a donc aussi pour objet l'utilisation d'au moins un dérivé de formule (I) comme agent exfoliant.

De plus, les dérivés selon l'invention se sont révélés particulièrement appropriés pour le soin et/ou le maquillage de la peau et/ou des muqueuses et/ou des fibres kératiniques d'être humain, et notamment pour lutter contre les signes du vieillissement cutané et/ou pour améliorer l'éclat du teint et/ou pour lisser la peau du visage et/ou du corps et/ou pour traiter les rides et les ridules de la peau et/ou pour stimuler le processus de renouvellement épidermique et/ou pour le traitement de l'acné et/ou pour le traitement des désordres cutanés.

Par désordres cutanés, on entend en particulier le zona, les brûlures, l'eczéma, la démodécie, l'ulcère cutané, la fibrose, le contrôle des cicatrisations, le psoriasis, les prurits, les dermatites, l'ichtyose, les cors et les verrues.

Aussi, l'invention a encore pour objet l'utilisation d'au moins un dérivé de formule (I) dans et/ou pour la fabrication d'une composition cosmétique et/ou dermatologique destinée au soin et/ou au maquillage de la peau et/ou des muqueuses et/ou des fibres kératiniques.

L'invention a aussi pour objet l'utilisation d'au moins un dérivé de formule (I) dans et/ou pour la fabrication d'une composition cosmétique et/ou dermatologique destinée à lutter contre les signes du vieillissement cutané et/ou à améliorer l'éclat du teint et/ou à lisser la peau du visage et/ou du corps et/ou à traiter les rides et les ridules de la peau et/ou à stimuler le processus de renouvellement épidermique.

L'invention a encore pour objet l'utilisation d'au moins un dérivé de formule (I) dans et/ou pour la fabrication d'une composition cosmétique et/ou dermatologique destinée à lutter contre l'acné.

L'invention a encore pour objet l'utilisation d'au moins un dérivé de formule (I) dans la fabrication d'une composition dermatologique destinée à lutter contre les désordres cutanés.

L'invention a encore pour objet un procédé de traitement cosmétique et/ou dermatologique des signes du vieillissement cutané, qui consiste à appliquer sur la peau présentant ces signes, au moins un dérivé de formule (I) dans un milieu physiologiquement acceptable.

L'invention a encore pour objet un procédé de traitement cosmétique et/ou dermatologique de l'acné consistant à appliquer sur la peau acnéique au moins un dérivé de formule (I), dans un milieu physiologiquement acceptable.

L'invention a enfin pour objet un procédé de traitement dermatologique des désordres cutanés, consistant à appliquer sur la peau au moins un dérivé de formule (I), dans un milieu physiologiquement acceptable.

D'autres caractéristiques et avantages de l'invention ressortiront mieux des exemples qui suivent, donnés à titre illustratif et non limitatif.

Les exemples suivants 1 et 2 illustrent le procédé de préparation des dérivés conformes à l'invention.

**Exemple 1 Préparation du 5-octanoyl-salicylate de strontium**

Dans un ballon de 500 ml muni d'un agitateur, d'un thermomètre et d'un réfrigérant ascendant, on introduit 39,6 g d'acide 5-octanoyl-salicylique (0,15 mole) dans 250 ml d'isopropanol. Quand la dissolution est totale, on introduit par petites fractions, 7,5 g de carbonate de strontium (0,075 mole). Le milieu devient épais et laiteux ; on ajoute alors 50 ml d'eau et on tiédit à 40-45°C.

Dès que tout le carbonate de strontium est introduit, le milieu devient progressivement limpide. On concentre alors, à sec, à l'évaporateur rotatif. On obtient 43,9 g d'une poudre blanche.

**Analyse élémentaire :** conforme.

**Exemple 2 Préparation du 5-octanoyl-salicylate de calcium**

Dans un ballon de 500 ml, on introduit 39,6 g d'acide 5-octanoyl-salicylique (0,15 mole) dans 300 ml d'isopropanol et 150 ml d'eau. Puis on ajoute par petites fractions, 7,5 g de carbonate de calcium (0,075 mole). On laisse sous agitation à température ambiante pendant 3 heures, puis on concentre à sec sous vide à l'évaporateur rotatif.

On obtient 42,1 g d'une poudre blanche.

**Analyse élémentaire :** conforme.

**Exemple de formulation 1 : Lait pour la peau, à activité exfoliante**

- 5-octanoyl-salicylate de strontium 0,5 %
- BRIJ92 2,5 %
- BRIJ96 2,5 %
- Huile de vaseline 30 %
- Eau qsp 100 %

Le lait obtenu est doux et a une bonne propriété exfoliante pour la peau.

TEST : La demanderesse a constaté que la stimulation du processus de renouvellement épidermique consistant à éliminer les cellules superficielles de la peau entraînait un lissage des traits, un ravivage du teint et une réduction des rides et ridules. Aussi, un composé est d'autant plus efficace pour le traitement des signes du vieillissement qu'il a une bonne propriété exfoliante. Cette propriété a été mise en évidence en effectuant un test *in vitro* de détachement cellulaire.

Ce test *in vitro* a été réalisé sur kératinocytes en utilisant la composition de l'exemple 1. Le principe du test repose sur le fait que le détachement cellulaire induit la libération de cornéocytes. Le pouvoir de traitement du vieillissement du produit testé va être d'autant plus grand que le nombre de cornéocytes libérés sera important.

Le protocole du test a été le suivant : à partir de biopsies de peau, on a obtenu, par séparation de l'épiderme, les kératinocytes que l'on a dissociés par action enzymatique à la trypsine et mis en culture à la concentration de $2.10^5$ cellules/ml. La croissance et la différenciation des kératinocytes ont été obtenues par culture durant 10 à 20 jours en milieu spécifique.

Puis, après élimination du milieu de culture, on a ajouté le produit à tester et évalué l'activité du produit. Pour ce faire, on a réalisé deux prélèvements à T0 et T60, c'est-à-dire avant l'ajout du produit et 60 minutes après cet ajout, et on a analysé les prélèvements ainsi effectués au cytomètre de flux pour dénombrer la population de cornéocytes. Au cytomètre de flux, les populations de cornéocytes et de kératinocytes sont différenciées par traitement à l'acridine orange spécifique de l'ADN des cellules, qui se lie au noyau des cellules et révèle donc exclusivement la présence des kératinocytes.

L'indice de détachement cellulaire est déterminé par la différence entre T60 et T0.

La même mesure a été réalisée comparativement à l'acide correspondant sous forme libre dans une composition analogue. La variation de cet acide a fixé arbitrairement la norme de 100 %.

Les résultats sont rassemblés dans le tableau ci-dessous :

| Acide 5-octanoyl-salicylique $5.10^{-5}$ M | Dérivé de l'exemple 1 $5.10^{-5}$ M |
|---|---|
| 100 % | 104.5 % |

Ce résultat montre que le dérivé selon l'invention favorise le détachement cellulaire au moins autant voire plus que l'acide libre.

**Exemple de formulation 2 : Lait pour la peau, à activité exfoliante**

Une seconde composition est réalisée en remplaçant dans l'exemple de formulation 1, le 5-octanoyl-salicylate de strontium par le 5-octanoyl-salicylate de calcium.
Le lait obtenu permet un traitement en douceur des signes du vieillissement de la peau.
Test : il a effectué un test sur cornée isolée pour mettre en évidence la diminution de l'irritation des exemples de formulation 1 et 2 par rapport à une composition analogue contenant de l'acide libre.
Ce test est basé sur la mesure de deux paramètres, l'opacité et la perméabilité, dont les altérations reflètent l'atteinte du tissu et donc le potentiel d'agressivité du produit sur la cornée isolée.
Les compositions étudiées sont mises en contact pendant 30 minutes dans des compartiments contenant de la cornée isolée. On mesure ensuite à l'aide d'un opacimètre, l'opacité des cornées traitées et celle d'une cornée témoin pour déterminer la différence de transmission entre la cornée témoin et les cornées traitées. Cette différence de transmission est mesurée juste après l'incubation et 2 heures après la première mesure ; c'est cette dernière valeur T qui est prise en compte.
Par ailleurs, on mesure également la perméabilité cornéenne juste après la deuxième mesure d'opacité : le compartiment contenant les cornées est rempli d'un milieu nuritif auquel on ajoute une solution de fluorescéine à 4 mg/ml et, après un temps de contact de 10 minutes, on prélève le milieu pour en déterminer la densité optique (DO) au spectrophotomètre à 490 nm.
Le score cornéen est calculé à partir de ces deux mesures selon le calcul suivant :

$$\text{Score cornéen} = T + 15\ DO$$

Le score cornéen est d'autant plus faible que l'irritation est moindre.
Les résultats du test sont indiqués dans le tableau 1 suivant :

Tableau 1

| **TESTS à 2 %** | **Score cornéen** |
|---|---|
| Exemple 1 | 11,9 ± 3 |
| Exemple 2 | 8,6 ± 3,8 |
| Acide 5-octanoyl salicylique à 0,5 % | 23,5 ± 2 |
| Témoin | 2,4 ± 1,9 |

Les différences significatives entre les exemples selon l'invention et l'acide 5-octanoyl-salicylique montrent une amélioration notable de la tolérance avec les dérivés selon l'invention par rapport à l'acide libre.
Par ailleurs, un autre test (figure 1) montre que les sels des exemples 1 et 2, appliqués sur un épiderme reconstruit (Episkin[R]) présentent une toxicité cellulaire significativement moins élevée que les associations correspondantes de l'acide libre et d'un sel comportant le même cation ou que l'acide libre seul.
La figure 1 montre le pourcentage de viabilité des cellules en fonction de la concentration p/v des produits testés. De gauche à droite, le premier histogramme est relatif au véhicule seul, les 4 histogrammes suivants sont relatifs à l'acide libre, les 4 suivants sont relatifs à l'association de l'acide libre et d'un sel de cation divalent, le cation divalent étant celui du dérivé selon l'invention de l'exemple 2, les 4 histogrammes suivants sont relatifs à un dérivé selon l'invention (exemple 2), les 4 histogrammes suivants sont relatifs à l'association de l'acide libre et d'un sel de cation divalent, le cation divalent étant celui du dérivé selon l'invention de l'exemple 1, les 4 derniers histogrammes sont relatifs à un dérivé selon l'invention (exemple 1).
Plus l'histogramme est élevé, plus le nombre de cellules en vie au bout de 18 heures est grand.

Les résultats obtenus ont été confirmés sur 3 lots différents d'épiderme humain reconstruit.

## Revendications

1. Dérivé de l'acide salicylique de formule (I) :

$$AR^{-}\ S^{2+}\ AR^{-} \qquad (I)$$

dans laquelle :

$S^{2+}$ représente un cation inorganique divalent ;
$AR^{-}$ représente le radical de formule (II) :

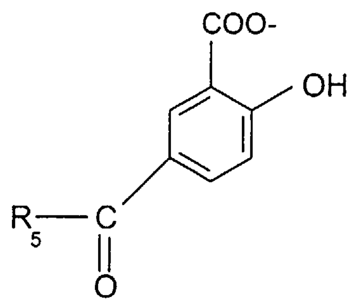

(II)

dans laquelle :
$R_5$ représente une chaîne aliphatique, linéaire ou ramifiée, saturée ou insaturée, ayant de 3 à 15 atomes de carbone.

2. Dérivé selon la revendication 1, caractérisé en ce qu'il est choisi parmi les sels des acides n-octanoyl-5-salicylique, n-décanoyl-5-salicylique, n-dodécanoyl-5-salicylique.

3. Dérivé selon l'une quelconque des revendications 1 et 2, caractérisé en ce que $S^{2+}$ représente un cation choisi parmi les éléments des colonnes IB, IIA, IIB, IIIB, IIIA, IVB, VB, VIB, VIIB, VIII de la classification périodique.

4. Dérivé selon l'une quelconque des revendications 1 à 3, caractérisé en ce que $S^{2+}$ représente un cation choisi parmi les cations des éléments des colonnes IB, IIA, IIB, VIIB, VIII.

5. Dérivé selon l'une quelconque des revendications 1 à 4, caractérisé en ce que $S^{2+}$ représente un cation choisi parmi les cations des éléments des colonnes IIA, IIB et VIIB de la classification périodique.

6. Dérivé selon l'une quelconque des revendications 1 à 5, caractérisé en ce que $S^{2+}$ représente un cation choisi parmi les cations de strontium, de calcium, de magnésium, de baryum et de manganèse.

7. Dérivé selon l'une quelconque des revendications 1 à 6, caractérisé en ce qu'il est choisi parmi le sel de strontium de l'acide 5-octanoyl salicylique, le sel de calcium de l'acide 5-octanoyl salicylique, le sel de magnésium de l'acide 5-octanoyl salicylique.

8. Procédé de préparation d'un dérivé selon l'une quelconque des revendications 1 à 7, consistant à faire réagir un dérivé de l'acide salicylique ayant la formule (III) :

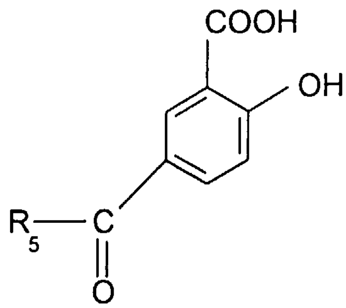

(III)

dans laquelle :
$R_5$ représente une chaîne aliphatique, linéaire ou ramifiée, saturée ou insaturée, ayant de 3 à 15 atomes de carbone, avec un composé comportant un cation inorganique divalent en présence d'un solvant.

**9.** Procédé selon la revendication 8, caractérisé en ce que le composé de cation divalent est choisi parmi les carbonates, bicarbonates, sulfates, glycérophosphates, borates, chlorures, nitrates, acétates, hydroxydes, persulfates, les sels d'α-hydroxy-acides ou d'acides de fruits, les sels d'acides aminés, les sels d'acides gras.

**10.** Procédé selon la revendication 8 ou 9, caractérisé en ce que le solvant est choisi parmi les alcools comportant de 1 à 12 atomes de carbone, les hydrocarbures, les cétones et l'eau, les éthers et les amines.

**11.** Procédé selon l'une quelconque des revendications 8 à 10, caractérisé en ce que le solvant est l'isopropanol.

**12.** Composition, caractérisée en ce qu'elle contient au moins un dérivé selon l'une quelconque des revendications 1 à 7.

**13.** Composition selon la revendication 12, caractérisée en ce qu'elle consiste en une composition topique.

**14.** Composition selon la revendication 12 ou 13, caractérisée en ce qu'elle constitue une composition cosmétique et/ou dermatologique.

**15.** Composition selon l'une quelconque des revendications 12 à 14, caractérisée en ce que le dérivé de formule (I) est présent en une quantité allant de 0,001 à 30 % du poids total de la composition, et de préférence de 0,01 à 20 % du poids total de la composition.

**16.** Composition selon l'une quelconque des revendications 12 à 15, caractérisée en ce qu'elle comprend, en outre, au moins un adjuvant choisi parmi l'eau, les corps gras, les conservateurs, les gélifiants, les tensioactifs et émulsionnants, les antioxydants, les charges, les solvants, les parfums, les matières colorantes, les filtres, les actifs cosmétiques et/ou dermatologiques et leurs mélanges.

**17.** Utilisation d'au moins un dérivé selon l'une quelconque des revendications 1 à 7 comme agent exfoliant.

**18.** Utilisation d'au moins un dérivé selon l'une quelconque des revendications 1 à 7, dans et/ou pour la fabrication d'une composition cosmétique et/ou dermatologique destinée au soin et/ou au maquillage de la peau et/ou des muqueuses et/ou des fibres kératiniques.

**19.** Utilisation d'au moins un dérivé selon l'une quelconque des revendications 1 à 7, dans et/ou pour la fabrication d'une composition cosmétique et/ou dermatologique destinée à lutter contre les signes du vieillissement cutané et/ou à améliorer l'éclat du teint et/ou à lisser la peau du visage et/ou du corps et/ou à traiter les rides et les ridules de la peau et/ou à stimuler le processus de renouvellement épidermique.

**20.** Utilisation d'au moins un dérivé selon l'une quelconque des revendications 1 à 7, dans et/ou pour la fabrication d'une composition cosmétique et/ou dermatologique destinée à lutter contre l'acné.

**21.** Utilisation d'au moins un dérivé selon l'une quelconque des revendications 1 à 7, dans et/ou pour la fabrication

d'une composition cosmétique et/ou dermatologique destinée à lutter contre les désordres cutanés.

**22.** Procédé de traitement cosmétique des signes du vieillissement de la peau, caractérisé en ce qu'il consiste à appliquer sur la peau présentant ces signes, au moins un dérivé selon l'une quelconque des revendications 1 à 7, dans un milieu physiologiquement acceptable.

**23.** Procédé de traitement cosmétique de l'acné consistant à appliquer sur la peau acnéique au moins un dérivé selon l'une quelconque des revendications 1 à 7, dans un milieu physiologiquement acceptable.

## Claims

**1.** Salicylic acid derivative of formula (I):

$$AR^{-}S^{2+}AR^{-} \quad (I)$$

in which:

$S^{2+}$ represents a divalent inorganic cation;
$AR^{-}$ represents the radical of formula (II) :

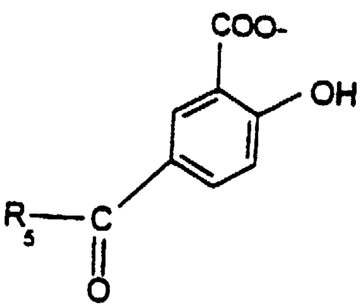

(II)

in which:
$R_5$ represents a linear or branched, saturated or unsaturated aliphatic chain having from 3 to 15 carbon atoms.

**2.** Derivative according to Claim 1, characterized in that it is chosen from salts of 5-n-octanoylsalicylic, 5-n-decanoylsalicylic and 5-n-dodecanoylsalicylic acid salts.

**3.** Derivative according to either of Claims 1 and 2, characterized in that $S^{2+}$ represents a cation chosen from the elements of columns IB, IIA, IIB, IIIB, IIIA, IVB, VB, VIB, VIIB and VIII of the Periodic Table.

**4.** Derivative according to any one of Claims 1 to 3, characterized in that $S^{2+}$ represents a cation chosen from the cations of the elements in columns IB, IIA, IIB, VIIB and VIII.

**5.** Derivative according to any one of Claims 1 to 4, characterized in that $S^{2+}$ represents a cation chosen from the cations of the elements in columns IIA, IIB and VIIB of the Periodic Table.

**6.** Derivative according to any one of Claims 1 to 5, characterized in that $S^{2+}$ represents a cation chosen from strontium, calcium, magnesium, barium and manganese cations.

**7.** Derivative according to any one of Claims 1 to 6, characterized in that it is chosen from the strontium salt of 5-octanoylsalicylic acid, the calcium salt of 5-octanoylsalicylic acid and the magnesium salt of 5-octanoylsalicylic acid.

**8.** Process for preparing a derivative according to any one of Claims 1 to 7, this process consisting in reacting a

salicylic acid derivative of formula (III):

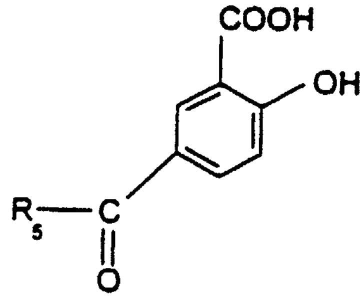

(III)

in which :
$R_5$ represents a linear or branched, saturated or unsaturated aliphatic chain having from 3 to 15 carbon atoms, with a compound containing a divalent inorganic cation, in the presence of a solvent.

**9.** Process according to Claim 8, characterized in that the divalent cation compound is chosen from carbonates, bicarbonates, sulphates, glycerophosphates, borates, chlorides, nitrates, acetates, hydroxides, persulphates, salts of á-hydroxy acids or fruit acids, salts of amino acids and salts of fatty acids.

**10.** Process according to Claim 8 or 9, characterized in that the solvent is chosen from alcohols containing from 1 to 12 carbon atoms, hydrocarbons, ketones, water, ethers and amines.

**11.** Process according to any one of Claims 8 to 10, characterized in that the solvent is isopropanol.

**12.** Composition, characterized in that it contains at least one derivative according to any one of Claims 1 to 7.

**13.** Composition according to Claim 12, characterized in that it consists of a topical composition.

**14.** Composition according to Claim 12 or 13, characterized in that it constitutes a cosmetic and/or dermatological composition.

**15.** Composition according to any one of Claims 12 to 14, characterized in that the derivative of formula (I) is present in an amount ranging from 0.001 to 30% of the total weight of the composition, and preferably from 0.01 to 20% of the total weight of the composition.

**16.** Composition according to any one of Claims 12 to 15, characterized in that it also comprises at least one adjuvant chosen from water, fatty substances, preserving agents, gelling agents, surfactants, emulsifiers, antioxidants, fillers, solvents, fragrances, dyestuffs, screening agents and cosmetic and/or dermatological active agents, and mixtures thereof.

**17.** Use of at least one derivative according to any one of Claims 1 to 7, as an exfoliant.

**18.** Use of at least one derivative according to any one of Claims 1 to 7, in and/or for the manufacture of a cosmetic and/or dermatological composition intended to care for and/or make up the skin and/or mucous membranes and/or keratin fibres.

**19.** Use of at least one derivative according to any one of Claims 1 to 7, in and/or for the manufacture of a cosmetic and/or dermatological composition intended to combat the signs of ageing of the skin and/or to improve the radiance of the complexion and/or to make facial and/or body skin smooth and/or to treat wrinkles and fine lines on the skin and/or to stimulate the process of epidermal renewal.

**20.** Use of at least one derivative according to any one of Claims 1 to 7, in and/or for the manufacture of a cosmetic and/or dermatological composition intended for combating acne.

**21.** Use of at least one derivative according to any one of Claims 1 to 7, in and/or for the manufacture of a cosmetic

and/or dermatological composition intended for combating skin disorders.

**22.** Process for the cosmetic treatment of the signs of ageing of the skin, characterized in that it consists in applying at least one derivative according to any one of Claims 1 to 7, in a physiologically acceptable medium, to skin showing these signs.

**23.** Process for the cosmetic treatment of acne, which consists in applying at least one derivative according to any one of Claims 1 to 7, in a physiologically acceptable medium, to acneic skin.

**Patentansprüche**

**1.** Salicylsäurederivat der Formel (I):

$$AR^{-}\ S^{2+}\ AR^{-} \quad (I),$$

worin bedeuten:

$S^{2+}$ ein zweiwertiges anorganisches Kation, und
$AR^{-}$ die Gruppe der folgenden Formel (II):

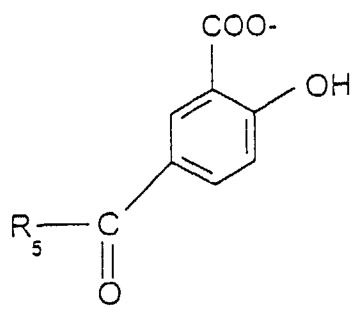

(II),

worin $R_5$ eine aliphatische, geradkettige oder verzweigte, gesättigte oder ungesättigte Gruppe mit 3 bis 15 Kohlenstoffatomen bedeutet.

**2.** Derivat nach Anspruch 1, dadurch gekennzeichnet, daß es unter den Salzen von 5-(n-Octanoyl)-salicylsäure, 5-(n-Decanoyl)-salicylsäure und 5-(n-Dodecanoyl)-salicylsäure ausgewählt ist.

**3.** Derivat nach einem der Ansprüche 1 oder 2, dadurch gekennzeichnet, daß $S^{2+}$ ein Kation bedeutet, das unter den Kationen der Elemente der Gruppen IB, IIA, IIB, IIIB, IIIA, IVB, VB, VIB, VIIB und VIII des Periodensystems ausgewählt ist.

**4.** Derivat nach einem der Ansprüche 1 bis 3, dadurch gekennzeichnet, daß $S^{2+}$ ein Kation bedeutet, das unter den Kationen der Elemente der Gruppen IB, IIA, IIB, VIIB und VIII ausgewählt ist.

**5.** Derivat nach einem der Ansprüche 1 bis 4, dadurch gekennzeichnet, daß $S^{2+}$ ein Kation bedeutet, das unter den Kationen der Elemente der Gruppen IIA, IIB und VIIB des Periodensystems ausgewählt ist.

**6.** Derivat nach einem der Ansprüche 1 bis 5, dadurch gekennzeichnet, daß $S^{2+}$ ein Kation bedeutet, das unter den Kationen von Strontium, Calcium, Magnesium, Barium und Mangan ausgewählt ist.

**7.** Derivat nach einem der Ansprüche 1 bis 6, dadurch gekennzeichnet, daß es unter dem Strontiumsalz von 5-Octanoylsalicylsäure, dem Calciumsalz von 5-Octanoyl-salicylsäure und dem Magnesiumsalz von 5-Octanoyl-salicylsäure ausgewählt ist.

**8.** Verfahren zur Herstellung von Derivaten nach einem der Ansprüche 1 bis 7, das darin besteht, ein Salicylsäurederivat der folgenden Formel (III)

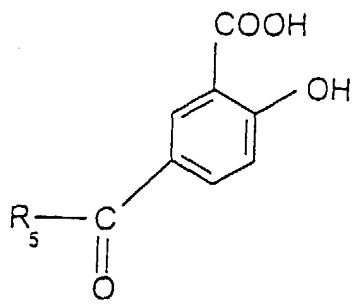

(III),

worin $R_5$ eine aliphatische, geradkettige oder verzweigte, gesättigte oder ungesättigte Gruppe mit 3 bis 15 Kohlenstoffatomen bedeutet,
mit einer Verbindung, die ein zweiwertiges anorganisches Kation enthält, in Gegenwart eines Lösungsmittels umzusetzen.

**9.** Verfahren nach Anspruch 8, dadurch gekennzeichnet, daß die Verbindung mit dem anorganischen Kation unter den Carbonaten, Bicarbonaten, Sulfaten, Glycerophosphaten, Boraten, Chloriden, Nitraten, Acetaten, Hydroxiden, Persulfaten, Salzen von $\alpha$-Hydroxysäuren oder Fruchtsäuren, Salzen von Aminosäuren und Salzen von Fettsäuren ausgewählt ist.

**10.** Verfahren nach Anspruch 8 oder 9, dadurch gekennzeichnet, daß das Lösungsmittel unter den Alkoholen mit 1 bis 12 Kohlenstoffatomen, Kohlenwasserstoffen, Ketonen und Wasser, Ethern und Aminen ausgewählt ist.

**11.** Verfahren nach einem der Ansprüche 8 bis 10, dadurch gekennzeichnet, daß das Lösungsmittel Isopropanol ist.

**12.** Zusammensetzung, dadurch gekennzeichnet, daß sie mindestens ein Derivat nach einem der Ansprüche 1 bis 7 enthält.

**13.** Zusammensetzung nach Anspruch 12, dadurch gekennzeichnet, daß es sich um eine topische Zusammensetzung handelt.

**14.** Zusammensetzung nach Anspruch 12 oder 13, dadurch gekennzeichnet, daß es sich um eine kosmetische und/oder dermatologische Zusammensetzung handelt.

**15.** Zusammensetzung nach einem der Ansprüche 12 bis 14, dadurch gekennzeichnet, daß das Derivat der Formel (I) in einem Anteil von 0,001 bis 30 % des Gesamtgewichts der Zusammensetzung und vorzugsweise von 0,01 bis 20 % des Gesamtgewichts der Zusammensetzung vorliegt.

**16.** Zusammensetzung nach einem der Ansprüche 12 bis 15, dadurch gekennzeichnet, daß sie ferner einen Zusatzstoff enthält, der unter Wasser, Fettsubstanzen, Konservierungsmitteln, Gelbildnern, grenzflächenaktiven Stoffen und Emulgatoren, Antioxidantien, Füllstoffen, Lösungsmitteln, Parfums, Färbemitteln, Filtern, kosmetischen und/oder dermatologischen Wirkstoffen und deren Gemischen ausgewählt ist.

**17.** Verwendung mindestens eines Derivats nach einem der Ansprüche 1 bis 7 als Exfoliationsmittel.

**18.** Verwendung mindestens eines Derivats nach einem der Ansprüche 1 bis 7 in einer kosmetischen und/oder dermatologischen Zusammensetzung und/oder zur Herstellung einer kosmetischen und/oder dermatologischen Zusammensetzung zur Pflege und/oder zum Schminken der Haut und/oder der Schleimhäute und/oder der Keratinfasern.

**19.** Verwendung mindestens eines Derivats nach einem der Ansprüche 1 bis 7 in einer kosmetischen und/oder dermatologischen Zusammensetzung und/oder zu deren Herstellung, um die Anzeichnen der Hautalterung zu bekämpfen und/oder den Teint strahlender erscheinen zu lassen und/oder die Haut des Gesichts und/oder des Körpers zu glätten und/oder die Falten und Fältchen der Haut zu behandeln und/oder den Prozeß der Erneuerung der Epidermis zu stimulieren.

**20.** Verwendung mindestens eines Derivats nach einem der Ansprüche 1 bis 7 in einer kosmetischen und/oder dermatologischen Zusammensetzung und/oder zu deren Herstellung zur Bekämpfung von Akne.

**21.** Verwendung mindestens eines Derivats nach einem der Ansprüche 1 bis 7 in einer kosmetischen und/oder dermatologischen Zusammensetzung und/oder zu deren Herstellung zur Behandlung von Hauterkrankungen.

**22.** Verfahren zur kosmetischen Behandlung von Anzeichen der Hautalterung, das darin besteht, mindestens ein Derivat nach einem der Ansprüche 1 bis 7 in einem physiologisch akzeptablen Medium auf die Haut, die diese Anzeichen zeigt, aufzubringen.

**23.** Verfahren zur kosmetischen Behandlung von Akne, das darin besteht, mindestens ein Derivat nach einem der Ansprüche 1 bis 7 in einem physiologisch akzeptablen Medium auf die von Akne betroffene Haut aufzubringen.

Figure 1

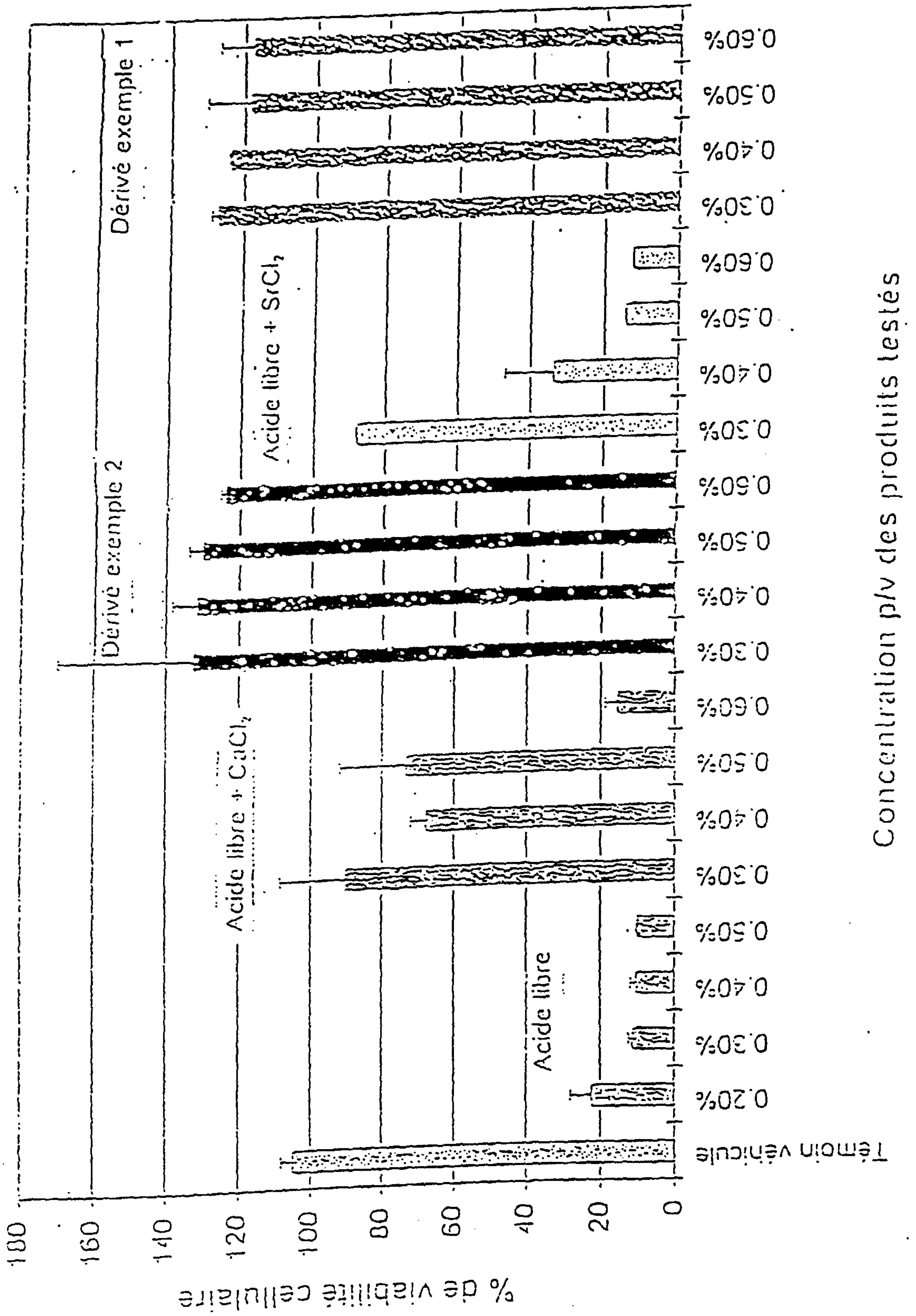
% de viabilité cellulaire
0
20
40
60
80
100
120
140
160
180
Témoin véhicule
0.20%
0.30%
0.40%
0.50%
0.60%
Acide libre
Acide libre + CaCl₂
Dérivé exemple 2
Acide libre + SrCl₂
Dérivé exemple 1
Concentration p/v des produits testés